(19) Europäisches Patentamt
European Patent Office
Office européen des brevets

(11) Veröffentlichungsnummer: **0 035 186**
**B1**

(12) # EUROPÄISCHE PATENTSCHRIFT

(45) Veröffentlichungstag der Patentschrift: **04.05.83**

(51) Int. Cl.³: **C 07 C 25/13,** C 07 C 17/12

(21) Anmeldenummer: **81101204.6**

(22) Anmeldetag: **20.02.81**

(54) **Verfahren zur Herstellung von 3-Brom-4-fluortoluol.**

(30) Priorität: **04.03.80 DE 3008158**

(43) Veröffentlichungstag der Anmeldung: **09.09.81 Patentblatt 81/36**

(45) Bekanntmachung des Hinweises auf die Patenterteilung: **04.05.83 Patentblatt 83/18**

(84) Benannte Vertragsstaaten: **AT BE CH DE FR GB IT LI NL**

(56) Entgegenhaltungen:
**DE-A-1 668 163**
**DE-A-1 923 656**

(73) Patentinhaber: **BAYER AG, Zentralbereich Patente, Marken und Lizenzen, D-5090 Leverkusen 1, Bayerwerk (DE)**

(72) Erfinder: **Baasner, Bernd, Dr., Kalkstrasse 132, D-5090 Leverkusen (DE)**
Erfinder: **Klauke, Erich, Dr., Eichendorffweg 8, D-5068 Odenthal (DE)**
Erfinder: **Kysela, Ernst, Dr., Virchowstrasse 14, D-5060 Bergisch Gladbach 3 (DE)**

EP 0 035 186 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von 3-Brom-4-fluortoluol

Die vorliegende Erfindung betrifft ein Verfahren zur Herstellung von 3-Brom-4-fluortoluol durch Bromierung von 4-Fluortoluol.

Es war bekannt, daß durch die Bromierung von 4-Fluortoluol in Tetrachlormethan mit Eisen als Katalysator (J. Ind. Chem. Soc. 21, 112 (1944)) oder durch Bromierung von 4-Fluortoluol in Substanz mit Aluminiumtribromid als Katalysator (Recl. Trav. Chim. Pays-bas 82, 9 (1963)) ein Gemisch der Isomeren 3-Brom-4-fluortoluol und 2-Brom-4-fluortoluol erhalten wird. Bei einer Nacharbeitung zeigte es sich, daß die Isomeren bei diesen Verfahren im Verhältnis von etwa 20 : 80 zueinander stehen. Darüber hinaus fallen bis zu 10% Dibrom-4-fluortoluole an. Der Nachteil bei diesen Reaktionsführungen ist daher der ungünstige Isomerenanteil von ca. 20% an 3-Brom-4-fluortoluol.

Es wurde nun gefunden, daß man bei der Bromierung von 4-Fluortoluol zu einer höheren Ausbeute an 3-Brom-4-fluortoluol dann gelangt, wenn man die Bromierung von 4-Fluortoluol in Eisessig als Lösungsmittel und in Anwesenheit von Jod und Eisenpulver beziehungsweise Eisensalzen durchführt.

Bei dieser Reaktion erhält man das 3-Brom-4-fluortoluol in einem Verhältnis von bis zu 70% gegenüber 30% des 2-Brom-4-fluortoluols. Dieses Isomerenverhältnis wird nur bei Verwendung des erfindungsgemäßen Katalysatorsystems erreicht.

Der Reaktionsverlauf des Verfahrens wird durch das folgende Formelschema wiedergegeben:

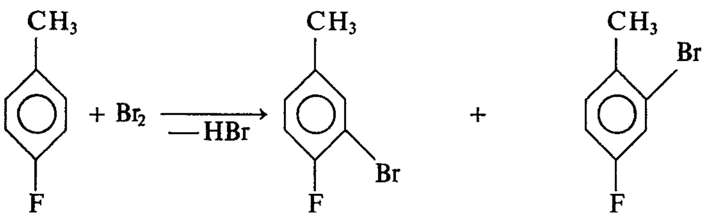


3-Brom-4-fluortoluol    2-Brom-4-fluortoluol

Bei der Durchführung des erfindungsgemäßen Verfahrens kann eine 30 bis 90%ige, vorzugsweise eine 70 bis 75%ige Lösung von 4-Fluortoluol in Eisessig mit 0,01 bis 10 Gew.-Prozent Eisenpulver, beziehungsweise mit 0,01 bis 10 Gew.-Prozent Eisensalzen, und 0,01 bis 10 Gew.-Prozent Jod, bezogen jeweils auf das Gewicht an eingesetztem 4-Fluortoluol, versetzt werden. Es ist vorteilhaft, das Eisenpulver durch Reduktion herzustellen.

Am vorteilhaftesten werden, bezogen auf 4-Fluortoluol, 0,05 bis 0,15 Gew.-Prozent Eisenpulver oder Eisensalze und 0,05 bis 0,15 Gew.-Prozent Jod verwendet.

Zu einem so bereiteten Gemisch gibt man im allgemeinen bei −10° C bis +50° C, vorzugsweise bei +20° C bis +30° C, eine 30 bis 95%ige, vorzugsweise eine 70 bis 75%ige Lösung von Brom in Eisessig. Das molare Verhältnis von 4-Fluortoluol zu Brom sollte im allgemeinen 1 zu 0,8 bis 1,5, vorzugsweise 1 zu 1 bis 1,1, am vorteilhaftesten jedoch 1 zu 1, betragen. Anschließend wird das Reaktionsgemisch im allgemeinen bei Temperaturen zwischen +20° C und +35° C 3 bis 18 Stunden bei Normaldruck nachgerührt. Bei sehr großem Ansatzvolumen ist es von Vorteil, diese Nachreaktionszeit zu verlängern.

Die günstigste Durchführungsart des erfindungsgemäßen Verfahrens besteht darin, die Lösung von Brom in Eisessig in einem Guß zu dem Gemisch aus Eisessig, 4-Fluortoluol und Katalysatoren zu geben, da somit die Bildung von Dibrom-4-fluortoluolen auf maximal 2% eingeschränkt bleibt. Darüber hinaus ist eine Nachrührzeit von bereits 3 bis 8 Stunden ausreichend, um einen relevanten Umsatz an 4-Fluortoluol zu erhalten.

Die Aufarbeitung des Reaktionsgemisches erfolgt destillativ. Das Rohgemisch kann bereits aus dem Reaktionsgefäß bei Normaldruck oder vermindertem Druck fraktioniert werden. Am vorteilhaftesten jedoch wird zunächst der gesamte destillierbare Anteil von einem geringen Rückstand, der sich überwiegend aus höher bromierten Toluolen zusammensetzt, im Vakuum abdestilliert. Unter Vermeidung von starken thermischen Belastungen des Rohgemisches können Neben- und Zersetzungsreaktionen somit weitgehend verhindert werden. Anschließend wird das Rohdestillat bei Normaldruck oder vermindertem Druck destillativ an einer Kolonne aufgetrennt. Die aus Lösungsmittel und nicht umgesetztem 4-Fluortoluol bestehende Vorfraktion kann erneut bei Bromierungsreaktionen des 4-Fluortoluols eingesetzt werden.

Bei einem Umsatz an 4-Fluortoluol von z. B. 40% erhält man ein Isomerenverhältnis von 3-Brom-4-fluortoluol zu 2-Brom-4-fluortoluol von (60 bis 70)% zu (40 bis 30)%. Eine Umsatzsteigerung gelingt einfach durch die Verlängerung der Reaktionszeit, das Isomerenverhältnis bleibt dabei unverändert.

Beispiel A (Literaturbekanntes Verfahren nach J. Ind. Chem. Soc. 21, 112 (1944))

Zu einer Lösung von 110 g (1 Mol) 4-Fluortoluol in 165 ml Tetrachlormethan gibt man 11 g Eisenpulver. Dazu tropft man bei Raumtemperatur 160 g (1 Mol) Brom, läßt 24 Stunden nachrühren, destilliert bei Normaldruck das Lösungsmittel ab und fraktioniert den Rückstand bei Normaldruck über eine 10-cm-Vigreux-Kolonne. Man erhält als erste Fraktion bei Kp. 114 – 117°C 26,4 g nicht umgesetztes 4-Fluortoluol, als zweite Fraktion bei Kp. 175 – 185°C 78 g eines Gemisches bestehend aus 66% 2-Brom-4-fluortoluol, 24% 3-Brom-4-fluortoluol und 9% Dibrom-4-fluortoluol. Bezogen auf umgesetztes 4-Fluortoluol beträgt die Ausbeute an 2-Brom-4-fluortoluol plus 3-Brom-4-fluortoluol 48%, die an 3-Brom-4-fluortoluol 11,4%.

Beispiel B (Literaturbekanntes Verfahren nach Recl. Trav. Chim. Pays-Bas 82, 9 (1963))

Zu 110 g 4-Fluortoluol, die auf – 15°C gekühlt sind, gibt man 2,67 g Aluminiumtribromid und 16,0 g Brom. Nach Ansteigen der Temperatur auf + 10°C wird mit Wasser hydrolysiert, das Gemisch mit Dichlormethan ausgeschüttelt, gewaschen und getrocknet. Nach Abdestillieren des Lösungsmittels und des Überschusses an 4-Fluortoluol erhält man bei Kp. 172 – 184°C (Normaldruck) 5,5 g eines Gemisches bestehend aus 88% 2-Brom-4-fluortoluol, 8% 3-Brom-4-fluortoluol und 4% Dibrom-4-fluortoluol. Bezogen auf umgesetztes 4-Fluortoluol beträgt die Ausbeute an 3-Brom-4-fluortoluol 2,3%.

Beispiel 1

Zu einer Lösung von 110 g 4-Fluortoluol in 40 ml Eisessig, der 1,1 g Eisenpulver und 1,1 g Jod zugesetzt sind, gibt man innerhalb von 3 Stunden eine Lösung von 160 g Brom in 60 ml Eisessig. Die anfangs exotherme Reaktion wird zunächst mit Wasserkühlung, dann mit Warmwasser auf 25°C bis 27°C gehalten. Es wird 8 Stunden bei gleicher Temperatur nachgerührt, dann bei Normaldruck bis 120°C der Eisessig und nicht umgesetztes 4-Fluortoluol, anschließend bei 20 mbar zwischen 65°C und 85°C ein Gemisch, bestehend aus 3-Brom-4-fluortoluol (62%), 2-Brom-4-fluortoluol (32%) und Dibrom-4-fluortoluol (5%) abdestilliert. Es verbleiben 34 g Rückstand. Die monobromierten Isomere werden an einer Kolonne destillativ aufgetrennt. Man erhält 12,7 g 2-Brom-4-fluortoluol (Kp. 176 – 8°C, $n_D^{20}$: 1.5260) und 24,5 g 3-Brom-4-fluortoluol (Kp. 184 – 5°C, $n_D^{20}$: 1,5303).

Beispiel 2

Zu einer Lösung von 110 g 4-Fluortoluol in 40 ml Eisessig, der 1,1 g Eisenpulver und 1,1 g Jod zugesetzt sind, gibt man in einem Guß eine Lösung von 160 g Brom in 60 ml Eisessig. Die anfangs exotherme Reaktion wird zunächst mit Wasserkühlung, dann mit Warmwasser auf 25°C bis 27°C gehalten. Es wird 3 Stunden bei gleicher Temperatur nachgerührt, dann im Vakuum der Eisessig und nicht umgesetztes 4-Fluortoluol, anschließend bei 20 mbar zwischen 65°C und 85°C ein Gemisch bestehend aus 3-Brom-4-fluortoluol (57%), 2-Brom-4-fluortoluol (41,9%) und Dibrom-4-fluortoluol (1,1%) abdestilliert. Es verbleiben 8 g Rückstand. Die monobromierten Isomere werden an einer Kolonne destillativ aufgetrennt. Man erhält 21,5 g 2-Brom-4-fluortoluol (Kp. 176 – 8°C) und 28,5 g 3-Brom-4-fluortoluol (Kp. 184 – 5°C).

In der nachfolgenden Tabelle sind die Isomerenverteilungen, wie sie nach den verschiedenen Verfahren anfallen, zusammengestellt:

| Verfahren nach Beispiel | Anteil (%) an 2-Brom-4-fluor-toluol | 3-Brom-4-fluor-toluol | Dibrom-4-fluor-toluol |
|---|---|---|---|
| A | 66 | 24 | 9 |
| B | 88 | 8 | 4 |
| 1 | 32 | 62 | 5 |
| 2 | 41.9 | 57 | 1.1 |

## Patentansprüche

1. Verfahren zur Herstellung von 3-Brom-4-fluortoluol durch Bromierung von 4-Fluortoluol, dadurch gekennzeichnet, daß man in Eisessig und in Anwesenheit von Jod und Eisenpulver beziehungsweise Eisensalzen arbeitet.

2. Verfahren gemäß Anspruch 1, dadurch gekennzeichnet, daß man in Gegenwart von Jod und Eisenpulver arbeitet.

## Claims

1. Process for the preparation of 3-bromo-4-fluorotoluene by bromination of 4-fluorotoluene, characterized in that the bromination is carried out in glacial acetic acid and the presence of iodine and iron powder or iron salts.

2. Process according to claim 1, characterized in that the bromination is carried out in the presence of iodine and iron powder.

## Revendications

1. Procédé de préparation du 3-bromo-4-fluorotoluène par bromation du 4-fluorotoluène, caractérisé en ce que l'on opère dans l'acide acétique glacial et en présence d'iode et de poudre de fer ou de sels de fer.

2. Procédé selon la revendication 1, caractérisé en ce que l'on opère en présence d'iode et de poudre de fer.